# EUROPEAN PATENT APPLICATION

(11) **EP 2 272 950 A2**
(43) Date of publication of application: **12.01.2011**
(21) Application number: 10182312.8
(22) Date of filing: 08.11.2005
(51) Int. Cl.: C12N 7/04, C12N 5/10, A61K 39/145, A61K 48/00

(54) **Defective influenza virus particles**

(30) Priority: 12.11.2004 US 626878 P; 28.06.2005 US 694431 P; 11.11.2004 EP 04105696; 27.06.2005 EP 05105708
(62) Divisional of application: 05801665.0
(71) Applicant: Abbott Biologicals B.V., 1381 CP Weesp (NL); Erasmus University Medical Center Rotterdam, 3015 GD Rotterdam (NL)
(72) Inventor: de Wit, Emmie, 1381CP Weesp (NL); Spronken, Monique I.J., 1381CP Weesp (NL); Fouchier, Ron A.M., 1381CP Weesp (NL); Osterhaus, Albert D.M.E., 1381CP Weesp (NL)
(74) Representative: Aerts, Robert Jaap

(57) **Abstract**

The invention relates to the field of influenza virus and the vaccination against flu. The invention provides a conditionally defective influenza virus particle having seven different influenza nucleic acid segments. The invention also provides a conditionally defective influenza virus particle lacking an influenza nucleic acid segment selected from the group of segments essentially encoding acidic polymerase (PA), the basic polymerase 1 (PB1) and the basic polymerase 2 (PB2). In particular, the invention provides defective influenza virus particles having seven different influenza nucleic acid segments and lacking an influenza nucleic acid segment essentially encoding acidic polymerase. Furthermore, the invention provides use of a composition comprising a defective influenza virus particle according to the invention for the production of a pharmaceutical composition directed at generating immunological protection against infection of a subject with an influenza virus, and provides a method for generating immunological protection against infection of a subject with an influenza virus comprising providing a subject in need thereof with a composition comprising such defective influenza virus particle.

## Description

The invention relates to the field of influenza virus and the vaccination against flu.

Influenza viruses (Orthomyxoviridae) are enveloped negative-strand RNA viruses with a segmented genome (Taubenberger and Layne, Molecular Diagnosis Vol. 6 No. 4 2001). They are divided into two genera: one including influenza A and B and the other consisting of influenza C, based on significant antigenic differences between their nucleoprotein and matrix proteins. The three virus types also differ in pathogenicity and genomic organization. Type A is found in a wide range of warm-blooded animals, but types B and C are predominantly human pathogens. Influenza A viruses are further subdivided by antigenic characterization of the hemagglutinin (HA) and NA surface glycoproteins that project from the surface of the virion. There are currently 15 HA and nine NA subtypes. Influenza A viruses infect a wide variety of animals, including birds, swine, horses, humans, and other mammals. Aquatic birds serve as the natural reservoir for all known subtypes of influenza A and probably are the source of genetic material for human pandemic influenza strains.

Unlike the related paramyxoviruses, influenza viruses have a segmented RNA genome. Influenza A and B viruses have a similar structure, whereas influenza C is more divergent. Where the A and B type viruses each contain eight discrete gene segments coding for at least one protein each, the C type contains seven discrete segments, combining segment 4 and 6 of the A and B types. Influenza A and B viruses are covered with projections of three proteins: HA, NA, and matrix 2 (M2). Influenza C virus has only one surface glycoprotein. Each influenza RNA segment is encapsidated by nucleoproteins (NP) to form ribonucleotidenucleoprotein (RNP) complexes. The three polymerase proteins are associated with one end of the RNP complex. RNPs are surrounded by a membrane with the matrix protein (matrix 1) as an integral part. The phospholipid portion of the envelope is derived from the cellular host membrane. Also found within the virus particle is nonstructural protein 2 (NS2).

World Health Organization (WHO) guidelines for nomenclature of influenza viruses are as follows. First, type of virus is designated (A, B, or C), then the host (if nonhuman), place of isolation, isolation number, and year of isolation (separated by slashes). For influenza A, HA and NA subtypes are noted in parentheses. For example, strains included in the recent trivalent vaccine for the 2000 to 2001 season are: A/Panama/2007/99 (H3N2), A/New Caledonia/20/99 (H1N1), and B/Yamanashi/16/98. Since 1977, there have been two influenza A subtypes co circulating in humans: H1N1 and H3N2.

Influenza viruses accumulate point mutations during replication because their RNA polymerase complex has no proofreading activity. Mutations that change amino acids in the antigenic portions of surface glycoproteins may give selective advantages for a viral strain by allowing it to evade preexisting immunity. The HA molecule initiates infection by binding to receptors on certain host cells. Antibodies against the HA protein prevent receptor binding and are very effective at preventing reinfection with the same strain. HA can evade previously acquired immunity by either antigenic drift, in which mutations of the currently circulating HA gene disrupt antibody binding, or antigenic shift, in which the virus acquires HA of a new subtype. Antigenic drift pressures are unequal across the HA molecule, with positively selected changes occurring predominantly on the globular head of the HA protein. These changes also accumulate to a greater extent in HA than NA. Changes in other influenza proteins occur more slowly. Likewise, antigenic drift pressure is greatest in human-adapted influenza strains, intermediate in swine- and equine-adapted strains, and least in avian-adapted strains.

Because influenza viruses have a segmented genome, co infection with two different strains in the same host can lead to the production of novel reassorted influenza strains containing different combinations of parental gene segments. Fifteen HA subtypes are known to exist in wild birds and provide a source of HA's that are novel to humans. The emergence in human circulation of an influenza strain with a novel subtype by antigenic shift has been the cause of the last two influenza pandemics in 1957 and 1968 and was most likely the cause of the 1918 influenza pandemic. To be concordant with all that is known about the emergence of pandemic influenza viruses, a pandemic strain must have an HA antigenically distinct from the one currently prevailing; this HA cannot have circulated in humans for 60 to 70 years; and the virus must be transmissible from human to human. In both 1957 and 1968, pandemics resulted from a shift in HA, and in both cases, HA's of pandemic strains were closely related to avian strains. Although one of the absolute requirements for a pandemic is that HA must change, the extent to which the rest of the virus can or must change is not known. Only the pandemic viruses of 1957 and 1968 are available for direct study, the 1918 pandemic influenza virus is being characterized using molecular archeology. In 1957, three genes were replaced by avianlike genes: HA, NA, and a subunit of the polymerase complex (PB1). In 1968, only HA and PB1 were replaced.

A specific diagnosis of influenza infection can be made by virus isolation, hemagglutination inhibition (HI) test, antigen detection by immunoassay, serological tests, demonstration of NA activity in secretions, or molecular-based assays. Specimens can be collected as sputum, nasopharyngeal swab, or nasopharyngeal washing obtained by gorgling a buffered-saline solution. The standard for influenza diagnosis has been immunologic characterization after culture. Serological analysis provides an accurate but retrospective method for influenza infection because it requires collection of both acute and convalescent sera.

Influenza viruses can be grown in embryonated hens' eggs or a number of tissue culture systems. The addition of trypsin (for the cleavage activation of HA) allows influenza virus propagation in Madin-Darby canine kidney (MDCK) cells and other lines. The primary method for vaccine production is still the cultivation of influenza viruses in eggs. Culture in cell lines is commonly used for the primary isolation of human influenza viruses (both types A and B). Many human influenza viruses can be cultivated directly in the allantoic cavity of embryonated eggs. Some influenza A and B viruses require initial cultivation in the amniotic cavity and subsequent adaptation to the allantoic cavity. After culture isolation, most influenza isolates are definitively identified using immunoassays or immunofluorescence. HA molecules of influenza viruses bind sialic acid residues on the surface of respiratory cells for the virus to gain entry.

Influenza strains can be characterized antigenically by taking advantage of the ability of influenza viruses to agglutinate erythrocytes *in vitro.* Anti-HA antibodies can inhibit agglutination. Thus, a haemagglutination inhibition (HI) assay is one of the standard methods used to characterize influenza strains. HI assays are used to determine whether sample strains are immunologically related (i.e., cross-reactive) to recent vaccine strains. Typing sera, generally produced in ferrets, are added to wells in a series of twofold dilutions, and laboratory workers score assay wells by looking for suspended versus clumped red blood cells. In most situations, a panel of sera is used for matching sample strains against vaccine and reference strains, and during any given influenza season, the vast majority of sample strains are successfully matched by HI assays. WHO provides guidelines and WHO Collaborating Centers provide guidance on the identification of antigenic characteristics of individual virus strains. Sample strains are categorized according to immunologic pedigrees, such as A/Moscow/10/99 (H3N2)-like, A/New Caledonia/ 20/99 (H1N1)-like, and B/Beijing/184/93-like viruses. For sample strains that fail characterization in HI assays, laboratory workers must inoculate them into ferrets to produce a strain-specific antiserum. When the new antiserum is ready, HI assays are performed again as described. If the new serum shows significant gaps in cross-reactivity (usually defined as a fourfold difference between sample and vaccine strains), it is incorporated into the routine laboratory panel and used to look for new epidemic strains. Thus, HI assays are extremely important in the influenza virus surveillance effort for vaccine strain selection and are the most commonly used methods to assess antigenic drift.

Influenza strains can be characterized genetically by sequence comparison of the individual gene segments, and again WHO guidelines and WHO Collaborating Centers provide guidance on the identification of the individual identity of the RNA segments comprising the influenza genome; the influenza A and B virus nucleic acid segments encoding the nucleoprotein (NP), the basic polymerase 1 (PB1), the basic polymerase 2 (PB2), the acid polymerase (PA), the haemagglutinin (HA), the neuraminidase (NA), the matrixproteins (M1 and M2) and the nonstructural protein (NS1 and NS2), and the influenza C virus nucleic acid segments encoding the nucleoprotein (NP), the basic polymerase 1 (PB1), the basic polymerase 2 (PB2), the haemagglutinin-neuraminidase like glycoprotein (HN), the matrix proteins (M1 and M2) and the nonstructural protein (NS1 and NS2). Requests for reference strains for antigenic analysis, for nucleic acid sequence comparison and for identifying vaccine viruses can be addressed to the WHO Collaborating Centre for Reference and Research on Influenza, 45 Poplar Road, Parkville, Victoria 3052, Australia (fax: +61 3 9389 1881, web site: http//www.influenza centre.org); the WHO Collaborating Centre for Reference and Research on Influenza, National Institute of Infectious Diseases, Gakuen 4-7-1, Musashi-Murayama, Tokyo 208-0011, Japan (fax: +81 42 5610812 or +81 42 5652498); the WHO Collaborating Center for Surveillance, Epidemiology and Control of Influenza, Centers for Disease Control and Prevention, 1600 Clifton Road, Mail stop G16, Atlanta, GA 30333, United States of America (fax: +1 404 639 23 34); or the WHO Collaborating Centre for Reference and Research on Influenza, National Institute for Medical Research, The Ridgeway, Mill Hill, London NW7 1AA, England (fax: +44 208 906 4477). Updated epidemiological information is available on WHO's web site at http://www.who.int/influenza and the geographical information system, FluNet, at http://www.who.int/flunet

Awareness of the impact of influenza and of the health and economic benefits of its prevention is increasing, and the past decade has seen the use and benefits of vaccination and a number of anti-influenza drugs rise considerably. As a result of longer life expectancy in many countries, many more people are at risk of complications, the burden on the health care systems during influenza epidemics is more widely acknowledged, and more frequent international travel has created opportunities for the spread of the virus, while the introduction of new products has increased options for prevention and treatment of the disease. About 50 countries have government-funded national influenza immunization programmes and the vaccine is available in many others. Specific recommendations for the use of the vaccine vary, but generally involve annual immunization for individuals of advanced age and those aged over 6 months who are at increased risk of severe illness because of a pre-existing chronic medical condition. In some countries, vaccine is used to reduce the spread of influenza to those at increased medical risk. Member States need to consider the benefit of influenza prevention activities in the context of their overall public health priorities.

Inactivated vaccines are classified into several types, depending on whether they contain whole virus particles, partially disrupted virus particles (split vaccines) or purified envelope antigens (subunit vaccines). Some subunit vaccines have been combined with an adjuvant or delivery system.

A few countries have licensed live attenuated influenza vaccines for certain target groups. Two different formulations of 1 vaccine have been used in healthy adults and children in the Russian Federation, and another live vaccine has been tested extensively. However, until live attenuated vaccines are more widely available, they are not yet generally recommended for influenza prevention.

Two classes of antiviral agents have been developed for prevention and treatment of influenza. The M2 inhibitors, amantadine and rimantadine, are limited to treatment of influenza A viruses and have also been reported to be effective in prevention of infection. While both products cause some side-effects, significant neurological side-effects are more common with amantadine. Neuraminidase inhibitors, such as zanamivir and oseltamivir, have recently been licensed for treatment of types A and B influenza in a number of countries, and have been reported to be effective for prophylaxis. Resistant mutants have been detected in patients receiving both classes of antiviral agent. While this is not currently considered an important public health problem, the situation may change if these drugs are used on a very large scale.

WHO maintains a global international surveillance program operated with the cooperation of 110 national influenza centers located in 82 countries and 4 WHO collaborating centers for influenza reference and research located in Atlanta (United States), London (United Kingdom), Melbourne (Australia) and Tokyo (Japan). These centres provide an early warning system for emerging strains with epidemic potential. This system is important because the efficacy of the influenza vaccines is reduced if they do not contain the strains currently circulating. WHO issues recommendations for vaccine composition, as can be found in the Weekly Epidemiological Record (for example see issue 9, 2004, 79, page 88 or http://www.who.int/wer) published by the World Health Organization, in February for vaccines used in the northern hemisphere and in September for vaccines used in the southern hemisphere. As influenza has less defined seasonal patterns in equatorial regions, epidemiological considerations will influence which of these recommendations (February or September) is appropriate for vaccines for use in equatorial countries.

The collaborating centers carry out antigenic and genetic analysis of influenza isolates submitted by the national centers. Where evidence of antigenic variation is observed, this is collated with epidemiological data to assess the epidemiological significance of variants. Representative isolates are compared with the current vaccine strains using panels of human sera collected prior to and after vaccination, to assess whether current vaccines could be expected to protect against these viruses. Following publication of WHO's annual vaccine recommendations, high growth strains are developed and provided to manufacturers as reference viruses to assist in the generation of seed viruses for vaccine production. Tests for safety and potency of influenza vaccines include virus inactivation, microbial sterility, measurement of chemicals used for disrupting the virus and confirmation of the recommended antigen concentration. It is recommended that vaccines should comply with WHO requirements, however, the national control authorities should approve the specific vaccine viruses used in each country. National public health authorities are responsible for recommendations regarding the use of the vaccine. Also WHO has published recommendations on the prevention of influenza (See WER No. 35, 2002, pp. 281-288.)

It has already been shown that current flu vaccines do not protect naive individuals, a fact that becomes of immediate importance in case of a pandemic outbreak of influenza when many individuals that have not encountered a flu infection before are then at risk. Viruses generally initiate their life cycle by attaching to host cell surface receptors, entering the cells, and uncoating their viral nucleic acid, followed by replication of the viral genome. After new copies of viral proteins and genes are synthesized, these components assemble into progeny virions, which then exit the cell. During the assembly step, the progeny virus must select its genomic nucleic acid efficiently from a large pool of viral and cellular nucleic acids present in the cytoplasm. The packaging of viral genomes into virions typically involves recognition by viral components of a cis-acting sequence in the viral nucleic acid, the so-called "packaging signal." Defining such signals is important for understanding the viral life cycle and provides us with information that could be used to construct viral vectors for the expression of foreign proteins. Indeed, the utility of retroviruses as vehicles for gene delivery vectors for the expression of foreign proteins can be attributed in large measure to the well-established knowledge of the process of their vRNA packaging into progeny virions.

The genomic packaging signals of other RNA viruses are poorly understood, impeding progress in their use as vectors for the expression and delivery of foreign genes. Influenza A virus for example is an enveloped negative-strand RNA virus whose segmented genome has a coding capacity for the nucleoprotein (NP), the basic polymerase 1 (PB1), the basic polymerase 2 (PB2), the acidic polymerase (PA), the haemagglutinin (HA), the neuraminidase (NA), the matrix proteins (M1 and M2) and the nonstructural protein (NS1 and NS2).

This virus has two membrane-spanning glycoproteins, haemagglutinin (HA) and neuraminidase (NA), on the envelope. The HA protein binds to sialic acid-containing receptors on the host cell surface and mediates fusion of the viral envelope with endosomal membrane after receptor-mediated endocytosis. In contrast, the NA protein plays a crucial role late in infection by removing sialic acid from sialyloligosaccharides, thus releasing newly assembled virions from the cell surface and preventing the self-aggregation of virus particles. Within the envelope, the viral genome, comprising eight different viral RNA (vRNA) segments, is tightly linked to the nucleoprotein (NP) and polymerase proteins (PA, PB1, and PB2), forming the ribonucleoprotein complexes. All eight (or in the case of C type virus: all seven) functional gene segments are required to produce infectious virus. Various mutations in the polymerase genes have been described (WO2004/094466, WO2003/091401, US 5578473, Fodor et al, J. Virol. 77,5017-5020, 2003) that change the polymerase activity or alter the polymerase in another way but do not make it loose its functionality in synthesizing viral RNA to render virus containing such mutated polymerases incapable of replication. In WO2004/094466, infectious virus with a mutated PA gene was produced, thereby showing the benefits of a selection system allowing producing and recovering infectious virus with mutated genes. In WO2003/091401, it is shown how to produce infectious virus with mutations in the polymerase genes to allow production and recovery of influenza virus with desirable properties relevant to live attenuated vaccine virus production, such as temperature sensitivity or other types of attenuation. In US 55788473, polymerase gene segments possibly altering the specificity and reducing the activity of various polymerases are suggested. These were however not used to reconstitute virus, let alone to reconstitute virus that has lost its polymerase activity altogether. Furthermore, in none of the above identified applications, defective particles that have lost their capacity to replicate are produced. It is well known that when influenza A viruses are passaged at a high multiplicity of infection, defective virus particles are generated that lack one or more functional gene segments. In such virus particles, one or more functional genes are replaced with defective interfering (DI) gene segments, due to errors made by the influenza virus polymerase. Due to the high multiplicity of infection, and hence infection of cells with more than 1 virus particle, the defects of viruses that contain DI RNA are complemented by viruses that contain intact copies of the missing functional genes. It was shown recently that certain mutations in the acidic polymerase gene could increase the efficiency of generation of virus particles with defective genes (Fodor 2003). It is important to note that the generation of defective virus particles in these experiments and the complementation both occur at random in such experiments. This random process limits the use of DI RNA and conditionally defective virus particles in practical applications. Moreover, when defective virus particles are produced using these published methods, wildtype replication-competent viruses are produced in addition to the desired conditionally defective viruses. Such replication competent viruses may either be fully wildtype (the helper virus) or reassortants resulting from genetic mixing of the helper virus with the defective virus. The packaging process of the gene segments of influenza virus, either through a random or a specific mechanism, has been under debate for many years. Pieces of evidence for both options have been described. Evidence for random packaging is that aggregated virus particles have a higher infectivity than non-aggregated virus particles and that when a cell culture is infected at a low mode of infection (moi), some infected cells lack the expression of one segment both suggesting that there are virions that do not contain the entire influenza virus genome. Further evidence of random packaging is that influenza viruses containing nine segments have been produced experimentally.

One argument for a specific packaging process is that although all gene segments are present in equal amounts in virus stocks, they are present in the producer cells in different amounts. Furthermore, when defective interfering (DI) particles are generated, the DI vRNA replaces the segment from which it is derived (A defective interfering particle is a virus particle in which one of the gene segments has a large internal deletion. These particles occur when virus is passaged at a high moi). Finally, the efficiency of virion formation increases with an increasing number of gene segments.

### Summary of the invention

The present invention provides the following aspects:
(1) A defective influenza virus particle lacking one functional influenza virus nucleic acid segment when compared to its natural genome.
(2) A defective influenza virus particle lacking a functional influenza virus nucleic acid segment encoding acidic polymerase (PA).
(3) A defective influenza virus particle according to item (1) wherein the segment that is lacking encodes acidic polymerase (PA).
(4) A defective influenza virus particle according to anyone of items (1) to (3) having the influenza virus nucleic acid segments encoding the viral glycoproteins.
(5) A defective influenza virus particle according to anyone of items (1) to (4) having the influenza virus nucleic acid segments encoding the nucleoprotein (NP), the basic polymerase 1 (PB1), the basic polymerase 2 (PB2), the haemagglutinin (HA), the neuraminidase (NA), the matrix proteins (M1 and M2) and the nonstructural protein (NS1 and NS2).
(6) A defective influenza virus particle according to anyone of items (1) to (5) having influenza virus nucleic acid segments that are derived from influenza virus that is recommended by WHO for vaccine purposes.
(7) A defective influenza virus particle according to anyone of items (1) to (5) having influenza virus nucleic acid segments that are derived from influenza A virus.
(8) A defective influenza virus particle according to anyone of items (1) to (7) provided with a nucleic acid not encoding an influenza peptide.
(9) A cell comprising a defective influenza virus particle according to anyone of items (1) to (8).
(10) A cell according to item (9) having been provided with influenza virus acid polymerase (PA)
(11) A composition comprising a defective influenza virus particle according to anyone of items (1) to (8) or a cell or material derived from a cell according to item (9) or (10).
(12) Use of a composition according to item (11) for the production of a pharmaceutical composition directed at generating immunological protection against infection of a subject with an influenza virus.
(13) A method for generating immunological protection against infection of a subject with an influenza virus comprising providing a subject in need thereof with a composition according to item (11).
(14) Use of a defective influenza virus particle according to item (8) for the production of a composition directed at delivery of a nucleic acid not encoding an influenza peptide to a cell.
(15) Use of a defective influenza virus particle according to item (8) for the production of a pharmaceutical composition directed at delivery of a nucleic acid not encoding an influenza peptide to a subject's cells.
(16) A method for delivery of a nucleic acid not encoding an influenza peptide to a cell comprising providing said cell with a defective influenza virus particle according to item (8).
(17) A method for delivery of a nucleic acid not encoding an influenza peptide to a subject comprising providing said subject with a defective influenza virus particle according to item (8).

Defective influenza virus particles (e.g. Mena I. et al., J. Virol. 70:5016-24 (1996); Neumann G. et al., J. Virol. 74:547-51 (2000).) may be useful as vaccine candidates because they will induce antibodies against other viral proteins besides HA and NA and, if they are able to enter the host cell, because they can induce cellular immune responses against the virus (e.g. helper T cells, cytotoxic T cells) in addition to humoral responses. So far, production of defective influenza virus particles has been achieved by transfection (Mena I. et al., J. Virol. 70:5016-24 (1996); Neumann G. et al., J. Virol. 74:547-51 (2000).), reducing the possibilities of producing large quantities of such particles. An alternative to this approach would be to produce virus particles that are *conditionally* defective, allowing them to replicate in a defined production system, but not in normal cells or production systems. To this end, cells of the production system would be modified to enable production of one or more of the influenza virus genes or gene products, allowing *trans-*complementation of a defective influenza virus particle. The present invention for the first time discloses *defined trans-*complementation of defective influenza virus particles. In the laboratory, *trans-*complementation of influenza virus particles has been observed when defective interfering influenza viruses are complemented in the same cells by viruses carrying the wild-type version of the defective interfering gene segment. This "natural system" of *trans-*complementation is not useful to produce defined conditionally defective influenza virus particles. First, this system requires complementation of one (partially) defective virus by at least one (partially) replication-competent virus that may result in the undesired production of fully infectious virus. Second, because the production of defective interfering particles occurs at random for the different gene segments, it is not possible to produce defined conditionally defective virus particles.

Conditionally defective influenza virus particles can theoretically be based on the deletion of entire gene segments or parts thereof. The ability to produce defined conditionally defective virus particles by deleting entire gene segments (and producing the encoded gene product(s) *in-trans)* would be limited if the packaging of the influenza virus genome relies on the presence of all 8 segments, which is an issue of much debate (see elsewhere in this description). If the packaging process requires the presence of all 8 gene segments, it is not known if all gene segments need to be present in a full length form, which complicates the production of conditionally defective virus particles even further. The present invention has solved these problems.

The invention provides a method for obtaining a conditionally defective influenza virus particle comprising a first step of transfecting a suitable first cell or cells such as a 293T cell with a gene construct having internal deletions, such as pΔPB2, pΔPB1, pΔPA or pDIPA as provided herein derived by internally deleting a nucleic acid encoding an influenza polymerase whereby said gene construct is incapable of producing a functional polymerase capable of copying or syntesizing viral RNA, and with complementing influenza virus nucleic acid segments encoding an influenza virus, such as the seven complementing constructs encoding A/WSN/33 (HW181-188, Hoffmann et al., 2000) and with an expression plasmid capable of expressing said polymerase in said cell, such as one of HMG-PB2, HMG-PB1, HMG-PA as provided herein and harvesting at least one virus particle from the supernatant of said first cell or cells at a suitable time point, such as within 10 to 50, preferably at around 20 to 30 hours after transfection; and a second step of transfecting a suitable second cell or cells such as a MDCK cell with an expression plasmid capable of expressing said polymerase in said cell; and a third step of transfecting said second cell or cells with supernatant comprising at least one virus particle obtained from said first cell; and a fourth step comprising harvesting at least one (now conditionally defective because the viruses produced lack a gene segment expressing a functional polymerase capable of copying or syntesizing viral RNA because they have packaged the gene segment with an internal deletion) virus particle from the supernatant of said first cell or cells at a suitable time point, such as from 24 to 96, preferably from 48 to 72 hours after transfection.

Preferred are internal deletions that render the gene segment incapable of producing a functional protein, but are not so large as to hinder packaging of the gene segments of the virus into viral particles. Preferably, these deletions as counted respectively from the 5' and 3' non-coding regions. For Influenza A, such preferred deletions start for example at a 5'-nucleotide situated between, but not encompassing, nucleotides 58 and 75, and finish at a 3'-nucleotide situated between, but not encompassing, nucleotides 27 and 50 for the PA protein, start at a 5'-nucleotide situated between, but not encompassing, nucleotides 43 and 75, and finish at a 3'-nucleotide situated between, but not encompassing, nucleotides 24 and 50 for the PB1 protein, start at a 5'-nucleotide situated between, but not encompassing, nucleotides 34 and 50, and finish at a 3'-nucleotide situated between, but not encompassing, nucleotides 27 and 50 for the PB2 protein. More preferably, these deletions: start at a 5'-nucleotide situated between, but not encompassing, nucleotides 58 and 100, and finish at a 3'-nucleotide situated between, but not encompassing, nucleotides 27 and 100 for the PA protein, start at a 5'-nucleotide situated between, but not encompassing, nucleotides 43 and 100, and finish at a 3'-nucleotide situated between, but not encompassing, nucleotides 24 and 100 for the PB1 protein, start at a 5'-nucleotide situated between, but not encompassing, nucleotides 34 and 100, and finish at a 3'-nucleotide situated between, but not encompassing, nucleotides 27 and 100 for the PB2 protein. Even more preferably, these deletions: start at a 5'-nucleotide situated between, but not encompassing, nucleotides 58 and 150, and finish at a 3'-nucleotide situated between, but not encompassing, nucleotides 27 and 150 for the PA protein, start at a 5'-nucleotide situated between, but not encompassing, nucleotides 43 and 150, and finish at a 3'-nucleotide situated between, but not encompassing, nucleotides 24 and 150 for the PB1 protein, start at a 5'-nucleotide situated between, but not encompassing, nucleotides 34 and 150, and finish at a 3'-nucleotide situated between, but not encompassing, nucleotides 27 and 150 for the PB2 protein. Yet even more preferably, these deletions: start at a 5'-nucleotide situated between, but not encompassing, nucleotides 58 and 175, and finish at a 3'-nucleotide situated between, but not encompassing, nucleotides 27 and 175 for the PA protein, start at a 5'-nucleotide situated between, but not encompassing, nucleotides 43 and 175, and finish at a 3'-nucleotide situated between, but not encompassing, nucleotides 24 and 175 for the PB1 protein, start at a 5'-nucleotide situated between, but not encompassing, nucleotides 34 and 175, and finish at a 3'-nucleotide situated between, but not encompassing, nucleotides 27 and 175 for the PB2 protein. Most preferably, these deletions: start at a 5'-nucleotide situated between, but not encompassing, nucleotides 58 and 207, and finish at a 3'-nucleotide situated between, but not encompassing, nucleotides 27 and 194 for the PA protein, start at a 5'-nucleotide situated between, but not encompassing, nucleotides 43 and 246, and finish at a 3'-nucleotide situated between, but not encompassing, nucleotides 24 and 197 for the PB1 protein, start at a 5'-nucleotide situated between, but not encompassing, nucleotides 34 and 234, and finish at a 3'-nucleotide situated between, but not encompassing, nucleotides 27 and 209 for the PB2 protein.

Herein, complementing segments are defined as the segments that lead to a complete set of the eight gene segments of for example influenza A virus. Thus, if segment 1 was already used to produce a defective segment, the complementing (non-defective) segments are segment 2, 3, 4, 5, 6, 7 and 8. If segment 2 is defective, the complementing segments are segment 1, 3, 4, 5, 6, 7 and 8. And so on. Advantageously, the invention produces a method whereby no helpervirus is required or present.

The invention provides an isolated and conditionally defective influenza virus particle lacking a functional influenza virus nucleic acid segment (herein also called a conditionally defective influenza virus particle) encoding a polymerase selected from the group acidic polymerase (PA), the basic polymerase 1 (PB1) and the basic polymerase 2 (PB2), said particle being incapable of generating or serving as a source to generate polymerase to copy or syntesize viral RNA thereby only and conditionally allowing generation of replicative virus particles in cells trans-complemented with a functional polymerase. Furthermore, the invention provides a method for obtaining a conditionally defective influenza virus particle comprising providing a cell by transcomplementation with a functional influenza virus polymerase.

In a preferred embodiment, a particle according to the invention replicates in a cell complemented with the analogous nucleic acid segment which is lacking in the particle itself, e.g. a particle lacking functional influenza virus nucleic acid PA segment replicates in a cell at least having been provided with a functional influenza virus nucleic acid PA segment, a particle lacking functional influenza virus nucleic acid PB1 segment replicates in a cell at least having been provided with a functional influenza virus nucleic acid PB1 segment, a particle lacking functional influenza virus nucleic acid PB2 segment replicates in a cell at least having been provided with a functional influenza virus nucleic acid PB segment, respectively. In a preferred embodiment, the invention provides a particle according to the invention having the influenza virus nucleic acid segments encoding the viral glycoproteins, more preferably having the influenza virus nucleic acid segments encoding the nucleoprotein (NP), the haemagglutinin (HA), the neuraminidase (NA), the matrix proteins (M1 and M2) and the nonstructural protein (NS1 and NS2). In one embodiment, a particle according to the invention is provided having influenza virus nucleic acid segments that are derived from influenza A virus. Also, a particle according to the invention is provided that is also provided with a nucleic acid not encoding an influenza peptide. Also, the invention provides an isolated cell comprising a particle according to the invention, said cell being free of wild type influenza virus or helper virus but preferably also having been provided or complemented with influenza virus polymerase or a gene segment encoding therefore. In a preferrered embodiment such cell is a trans-complemented 293T or MDCK cell. In one embodiment, the invention provides an isolated cell comprising a particle lacking functional influenza virus nucleic acid PA segment, said cell being free of wild type influenza virus or helper virus but at least having been provided or complemented with a functional influenza virus nucleic acid PA segment or functional PA. In another embodiment, the invention provides an isolated cell comprising a particle lacking functional influenza virus nucleic acid PB1 segment, said cell being free of wild type influenza virus or helper virus but at least having been provided with a functional influenza virus nucleic acid PB1 segment or functional PB1. In yet another embodiment, the invention provides an isolated cell comprising a particle lacking a functional influenza virus nucleic acid PB2 segment, said cell being free of wild type influenza virus or helper virus but at least having been provided or complemented with a functional influenza virus nucleic acid PB2 segment or functional PB2. Furthermore, the invention provides a composition comprising a particle according to the invention or a cell or material derived from a cell according to the invention, and use of such a composition for the production of a pharmaceutical composition directed at generating immunological protection against infection of a subject with an influenza virus. Herewith, the invention provides a method for generating immunological protection against infection of a subject with an influenza virus comprising providing a subject in need thereof with a composition according to the invention. Also, the invention provides use of an influenza virus particle according to the invention for the production of a composition directed at delivery of a nucleic acid not encoding an influenza peptide to a cell. Also, the invention provides use of a particle according to the invention for the production of a pharmaceutical composition directed at delivery of a nucleic acid not encoding an influenza peptide to a subject's cells, and a method for delivery of a nucleic acid not encoding an influenza peptide to a cell or subject comprising providing said cell or subject with a particle according to the invention.

The invention provides a conditionally defective influenza virus particle lacking one functional influenza virus segment when compared to its natural genome, that is: compared to wild type or helper A or B type virus, having seven (instead of eight) different functional influenza virus nucleic acid segments or compared to wild type or helper C type virus, having six (instead of seven) functional different influenza virus nucleic acid segments. When herein the term "conditionally defective" is used it includes, but is not limited to, viral particles wherein one of the gene segments of the virus has a large internal deletion that results in a non-functional protein being expressed from it. All eight gene segments of for example influenza A virus and all the proteins encoded by them are required for the production of infectious virus. A virus containing a defective gene segment is thus itself defective: it can infect a cell and can go through one round of replication because all viral proteins were present in the virion (this protein was for example produced by an expression plasmid when the virus was produced) but no infectious virus particles are produced in the infected cell because one of the viral proteins cannot be produced by the virus. However, when cells are infected that express the protein that is normally expressed by the defective gene segment, the defective virus can replicate in these cells because all viral proteins are present. Thus these viruses are conditionally defective: they cannot replicate unless a cell with the right condition is provided (in this case a cell expressing the viral protein that is not encoded by the virus because of the deletion in the gene segment).

Furthermore, the invention provides a conditionally defective influenza virus particle lacking a functional influenza virus nucleic acid segment encoding polymerase. Herein a functional influenza virus nucleic acid segment comprises a nucleic acid encoding a functional influenza protein that allows and is required for the generation of replicative virus. For example, influenza A virus is a negative strand RNA virus with an 8-segmented genome. The 8 gene segments encode 11 proteins; gene segments 1-8 encode basic polymerase 2 (PB2), basic polymerase 1 (PB1) and PB1-ORF2 (F2), acidic polymerase (PA), haemagglutinin (HA), nucleoprotein (NP), neuraminidase (NA), matrix proteins 1 and 2 (M1, M2) and non-structural proteins 1 and 2 (NS1, NS2) respectively. The coding regions of the 8 gene segments are flanked by non-coding regions (NCRs), which are required for viral RNA synthesis. The extreme 13 and 12 nucleotides at the 5' and 3'-ends of the viral genomic RNAs respectively, are conserved among all influenza A virus segments and are partially complementary, to form a secondary structure recognized by the viral polymerase complex. The NCRs may contain up to 60 additional nucleotides that are not conserved between the 8 gene segments, but are relatively conserved among different influenza viruses. The NCRs and flanking sequences in the coding regions may be required for efficient virus genome packaging. Thus a functional influenza virus nucleic acid segment consists of a sequence with coding potential for a functional influenza protein allowing the generation of replicative virus (1 or 2 open reading frames per segment), the NCRs required for transcription of mRNA, viral RNA (vRNA) and RNA complementary to the viral RNA (cRNA) and the packaging signal residing in the NCR and flanking coding sequences.

It is preferred that said conditionally defective influenza virus particle lacking one influenza virus nucleic acid lacks the segment that encodes functional polymerase, be it PA, PB1 or PB2. Furthermore, for vaccine purposes, it is preferred that said particle has the influenza virus nucleic acid segment(s) encoding the viral glycoprotein(s).

In one embodiment the invention provides an influenza A virus particle having seven different influenza A nucleic acid segments. The defective influenza virus particles according to the invention are capable of replication, albeit only once in suitable, albeit not complemented, host animals or cells. In suitably complemented cells, the particles according the invention can replicate more rounds. For vaccine and gene delivery purposes, it is a great advantage that the defective particles cannot indefinitely replicate in normal, not transcomplemented cells, thereby reducing the risk of spread of the vaccine virus from host to host and reducing the risk of reversion to wild-type virus.

This is the first time defective influenza A viruses are produced using reverse genetics that contain only seven functional gene segments and that can undergo one round of replication, or multiple rounds of replication when the defective gene segment is transcomplemented. In one embodiment, the invention provides a conditionally defective influenza virus particle lacking an influenza nucleic acid segment essentially encoding acidic polymerase (PA). Similar to transcomplementation of PA, trans-complementation of other influenza virus genes can be envisaged. However, since PA expression levels have been shown to be less critical as compared to expression levels of other influenza virus proteins, PA is the preferred gene segment of the polymerase group that is deleted, PB2 and PB1 deleted virus could be produced as well and NP deleted virus could not be transcomplemented. In a preferred embodiment, the invention provides a conditionally defective influenza A virus particle having seven different influenza A nucleic acid segments and lacking an influenza A nucleic acid segment essentially encoding acidic polymerase. For vaccine purposes, a preferred conditionally defective influenza A virus particle according to the invention has the influenza A nucleic acid segments essentially encoding the haemagglutinin (HA) and the neuraminidase (NA) proteins, these proteins being the most immunologically relevant for conferring protection. For selecting the appropriate gene segments for inclusion in a vaccine, it is preferred that gene segments are selected from a virus that is recommended by WHO for vaccine use. Of course, HA and NA subtypes can vary, depending on the HA and NA subtypes of the influenza variant against which one wants to vaccinate. It is most preferred to generate a conditionally defective influenza virus particle according to the invention which has the influenza A nucleic acid segments essentially encoding the nucleoprotein (NP), the basic polymerase 1 (PB1), the basic polymerase 2 (PB2), the haemagglutinin (HA), the neuraminidase (NA), the matrixproteins (M1 and M2) and the nonstructural protein (NS1 and NS2), essentially encoding herein in particular indicating that a functional protein is expressed from the respective gene segment. Such a particle is particularly provided in an isolated cell provided with functional PA or a functional gene segment encoding PA. In another embodiment a conditionally defective influenza virus particle according to the invention is herein provided which has the influenza A nucleic acid segments essentially encoding the nucleoprotein (NP), the acidic polymerase (PA), the basic polymerase 2 (PB2), the haemagglutinin (HA), the neuraminidase (NA), the matrixproteins (M1 and M2) and the nonstructural protein (NS1 and NS2), essentially encoding herein in particular indicating that a functional protein is expressed from the respective gene segment. Such a particle is particularly provided in an isolated cell provided with functional PB1 or a functional gene segment encoding PB1. In another embodiment a defective influenza virus particle according to the invention is herein provided which has the influenza A nucleic acid segments essentially encoding the nucleoprotein (NP), the acidic polymerase (PA), the basic polymerase 1 (PB1), the haemagglutinin (HA), the neuraminidase (NA), the matrixproteins (M1 and M2) and the nonstructural protein (NS1 and NS2), essentially encoding herein in particular indicating that a functional protein is expressed from the respective gene segment. Such a particle is particularly provided in an isolated cell provided with functional PB2 or a functional gene segment encoding PB2. In another embodiment, the invention provides particles according to the invention additional provided with a nucleic acid not encoding an influenza peptide, e.g., encoding a foreign protein or peptide useful for eliciting an immune response, or provided with a nucleic acid capable of interfering with a cell's or pathogen's functions in a cell.

Furthermore, the invention provides a cell comprising a influenza virus particle according to the invention. When the particle has not been provided with a gene segment essentially encoding the required polymerase, it is useful to consider a cell having been provided with suitably functional influenza virus polymerase, allowing multiple rounds of replication of the defective influenza virus particles in a thus complemented cell.

Also, the invention provides a composition comprising a defective influenza virus particle according to the invention or a cell or material derived from a cell according to the invention; such a composition can for example be used for the production of a pharmaceutical composition directed at generating immunological protection against infection of a subject with an influenza virus. Also, the invention provides a method for generating immunological protection against infection of a subject with an influenza virus comprising providing a subject in need thereof with such a composition. Besides the use of particles according to the invention as vaccine or immunogenic composition, such compositions are preferably formulated as a vaccine, i.e. by admixing viral particles, or viral proteins derived from such particles (split-vaccines) with an appropriate pharmaceutical carrier such as a salt solution or adjuvant (e.g. an aluminum salt or other excipient commonly used (see for example http://www.cdc.gov/nip/publications/pink/Appendices/A/Excipient.pdf.). The conditionally defective influenza virus particles according to the invention are also candidate vectors for foreign gene delivery and for expression of a foreign protein, since a functional gene can for example be inserted between the 5'and 3' PA sequences. Considering that the invention provides a method for obtaining a conditionally defective influenza virus particle, possibly provided with a foreign or host nucleic acid segment or fragment thereof, comprising a first step of transfecting a suitable first cell or cells, with one or more gene constructs derived by internally deleting a nucleic acid encoding an influenza protein whereby said gene constructs are incapable of producing a functional protein and do not hinder packaging of the gene segments of the virus into viral particles and with complementing influenza virus nucleic acid segments encoding an influenza virus, and with one or more expression plasmids capable of expressing said proteins in said cell, and harvesting at least one virus particle from the supernatant of said first cell or cells at a suitable time point after transfection; and a second step of transfecting a suitable second cell or cells with one or more expression plasmids capable of expressing said proteins in said cell; and a third step of infecting said second cell or cells with supernatant comprising at least one virus particle obtained from said first cell; and a fourth step comprising harvesting at least one virus particle from the supernatant of said second cell or cells at a suitable time point after infection. Herewith the invention provides a method for obtaining a conditionally defective influenza virus particle comprising the step of transfecting a suitable cell or cells, with one or more gene constructs derived by internally deleting a nucleic acid encoding an influenza polymerase whereby said gene constructs are incapable of producing a functional polymerase, but do not hinder packaging of the gene segments of the virus into viral particles and with complementing influenza virus nucleic acid segments encoding an influenza virus, and with one or more expression plasmids capable of expressing said polymerases in said cell, and harvesting at least one virus particle from the supernatant of said cell or cells at a suitable time point after infection. Said method for obtaining a conditionally defective influenza virus particle comprises a first step of transfecting a suitable cell or cells with one or more expression plasmids capable of expressing influenza polymerases in said cell; and a second step of infecting said cell or cells with supernatant comprising conditionally defective influenza virus particles; and a third step comprising harvesting at least one virus particle from the supernatant of said cell or cells at a suitable time point after infection, or a method for obtaining a conditionally defective influenza virus particle comprising a first step of transfecting a suitable first cell or cells, with one or more gene constructs derived by internally deleting a nucleic acid encoding an influenza polymerase whereby said gene constructs are incapable of producing a functional polymerase, but do not hinder packaging of the gene segments of the virus into viral particles and with complementing influenza virus nucleic acid segments encoding an influenza virus, and with one or more expression plasmids capable of expressing said polymerases in said cell, and harvesting at least one virus particle from the supernatant of said first cell or cells at a suitable time point after transfection; and a second step of transfecting a suitable second cell or cells with one or more expression plasmids capable of expressing said polymerases in said cell; and a third step of infecting said second cell or cells with supernatant comprising at least one virus particle obtained from said first cell; and a fourth step comprising harvesting at least one virus particle from the supernatant of said second cell or cells at a suitable time point after infection. In the methods, the said polymerases can be for instance acidic polymerase (PA), basic polymerase 1 (PB1) or basic polymerase 2 (PB2). Preferably, the invention provides a method whereby the internal deletion results from internally deleting a nucleic acid encoding an influenza polymerase which starts at a 5'-nucleotide situated between, but not encompassing, nucleotides 58 and 207 counted from the non-coding region, and finishes at a 3'-nucleotide situated between, but not encompassing, nucleotides 27 and 194 counted from the non-coding region for the PA protein, alternatively starts at a 5'-nucleotide situated between, but not encompassing, nucleotides 43 and 246 counted from the non-coding region, and finishes at a 3'-nucleotide situated between, but not encompassing, nucleotides 24 and 197 counted from the non-coding region for the PB1 protein, alternatively starts at a 5'-nucleotide situated between, but not encompassing, nucleotides 34 and 234 counted from the non-coding region, and finishes at a 3'-nucleotide situated between, but not encompassing, nucleotides 27 and 209 counted from the non-coding region for the PB2 protein. In another variant, a foreign fragment is inserted into this internal deletion. Furthermore, the invention provides a method whereby the cell or cells to be infected with supernatant comprising conditionally defective influenza virus particles already express the non-functional polymerases, such as a acidic polymerase (PA), basic polymerase 1 (PB1) or basic polymerase 2 (PB2), and influenza particles obtainable by a method as provided herein. It is for example herein provided that cell or cells to be transfected with the gene constructs and nucleic acid segments already express the non-functional polymerases. In particular, the invention provides an influenza virus particle comprising one or more nucleic acid segments with an internal deletion in the segment rendering the segment incapable of producing a functional influenza polymerase, but not hindering packaging of the gene segment of the virus into viral particles, whereby the polymerase is selected from the group of acidic polymerase (PA), basic polymerase 1 (PB1) or basic polymerase 2 (PB2). It is preferred that the internal deletion: starts at a 5'-nucleotide situated between, but not encompassing, nucleotides 58 and 207 counted from the non-coding region, and finishes at a 3'-nucleotide situated between, but not encompassing, nucleotides 27 and 194 counted from the non-coding region for the PA protein, starts at a 5'-nucleotide situated between, but not encompassing, nucleotides 43 and 246 counted from the non-coding region, and finishes at a 3'-nucleotide situated between, but not encompassing, nucleotides 24 and 197 counted from the non-coding region for the PB1 protein, starts at a 5'-nucleotide situated between, but not encompassing, nucleotides 34 and 234 counted from the non-coding region, and finishes at a 3'-nucleotide situated between, but not encompassing, nucleotides 27 and 209 counted from the non-coding region for the PB2 protein. In a preferred embodiment, the invention provides a particle according to the invention having the influenza virus nucleic acid segments encoding the viral glycoproteins. The invention also provides a particle according to the invention having the influenza virus nucleic acid segments encoding the nucleoprotein (NP), the basic polymerase 1 (PB1), the basic polymerase 2 (PB2), the haemagglutinin (HA), the neuraminidase (NA), the matrix proteins (M1 and M2) and the nonstructural protein (NS1 and NS2), or a particle having the influenza virus nucleic acid segments encoding the nucleoprotein (NP), the acid polymerase (PA), the basic polymerase 2 (PB2), the haemagglutinin (HA), the neuraminidase (NA), the matrix proteins (M1 and M2) and the nonstructural protein (NS1 and NS2), or a particle having the influenza virus nucleic acid segments encoding the nucleoprotein (NP), the acid polymerase (PA), the basic polymerase 1 (PB1), the haemagglutinin (HA), the neuraminidase (NA), the matrix proteins (M1 and M2) and the nonstructural protein (NS1 and NS2). In particular the invention provides a particle according to the invention having influenza virus nucleic acid segments that are derived from influenza A virus. The invention also provides a particle according to the invention provided with a nucleic acid not encoding an influenza peptide. Furthermore, the invention provides a cell comprising a particle according to the invention, in particular a cell having been provided with one or more influenza virus polymerases whereby the polymerase is selected from the group of acidic polymerase (PA), basic polymerase 1 (PB1) or basic polymerase 2 (PB2). In addition, the invention provides a composition comprising a particle according to the invention or a cell or material derived from a cell according to the invention, the use of such a composition for the production of a pharmaceutical composition directed at generating immunological protection against infection of a subject with an influenza virus, and a method for generating immunological protection against infection of a subject with an influenza virus comprising providing a subject in need thereof with such a composition. Furthermore, the invention provides use of a particle according to the invention for the production of a composition directed at delivery of a nucleic acid not encoding an influenza peptide to a cell, and use of a particle according to the invention for the production of a pharmaceutical composition directed at delivery of a nucleic acid not encoding an influenza peptide to a subject's cells. Such a nucleic acid (herein also called a foreign nucleic acid) may encode a foreign gene or gene fragment encoding a suitable antigenic epitope or protein, or may encode a stretch of nucleotides capable of interfering with nucleic acid transcription in a cell. In one embodiment, the invention provides use of an influenza A virus particle according to the invention for the production of a composition directed at delivery of a nucleic acid not encoding an influenza peptide to a cell or a subject's cell. Furthermore, the invention provides a method for delivery of a nucleic acid not encoding an influenza peptide to a cell or a subject comprising providing said cell or said subject with a defective influenza virus particle provided with a foreign nucleic acid according to the invention.

### Figure legends

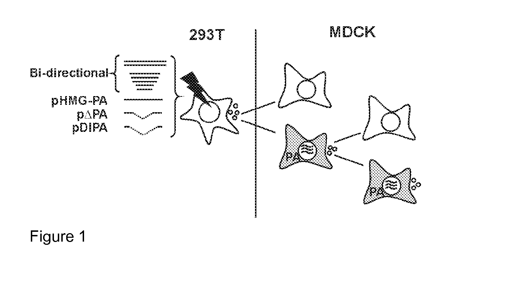

### Legend with figure 1

The production and propagation of conditionally defective influenza A virus. First, 293T cells were transfected with 7 bi-directional plasmids encoding A/PR/8/34, pHMG-PA and, if appropriate, pAPA or pDIPA. 48 hours after transfection, supernatants of transfected cells were harvested and used to inoculate MDCK cells and MDCK cells transfected with HMG-PA 24h earlier. The supernatant of the MDCK-PA cells positive for virus replication was passaged on MDCK and MDCK-PA cells 4 times.

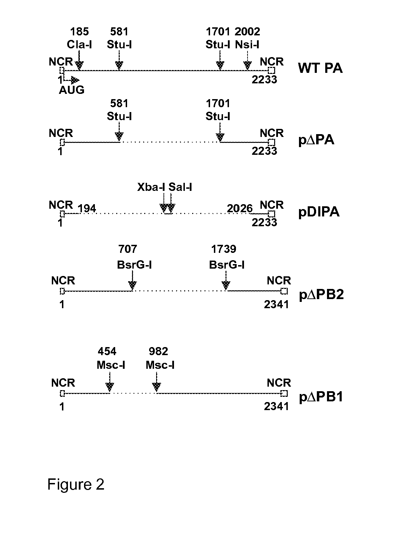

### Legend with figure 2

Constructs used for generating conditionally defective virus particles. The top shows a wild type PA gene segment. Non-coding regions (NCRs), and initiation codons are indicated. pΔPA was constructed by digestion of pHW183, a bi-directional plasmid containing PA of A/WSN/33 (9) with Stul and subsequent religation. pDIPA was constructed by cloning the 5' 194 and 3' 207 nts of the PA gene segment of A/PR/8/34 in pSP72. The insert was then transferred to a bi-directional reverse genetics vector. pΔPB1 and pΔPB2 were constructed as described in the text.

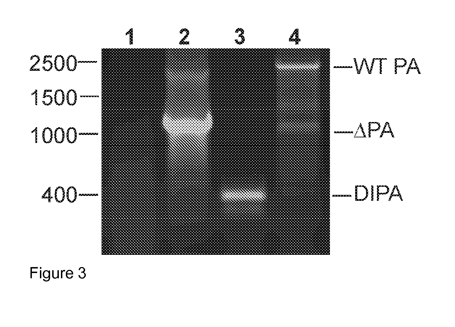

### Legend with figure 3

RT-PCR analysis for the presence of the PA gene segment in supernatants rPR8-7, rPR8-ΔPA and rPRB-DIPA. MDCK-PA passage 4 supernatants were passed through a 22µM filter and concentrated by centrifugation. Subsequently, RNA was isolated and a RT-PCR was performed using primers directed to the non-coding regions of the PA segment. RNA isolated from wild type A/PR/8/34 was used as a control. Lane 1: rPR8-7; lane 2: rPR8-ΔPA; lane 3 rPR8-DIPA; lane 4: wild-type A/PR/8/34. Marker sizes are indicated on the left.

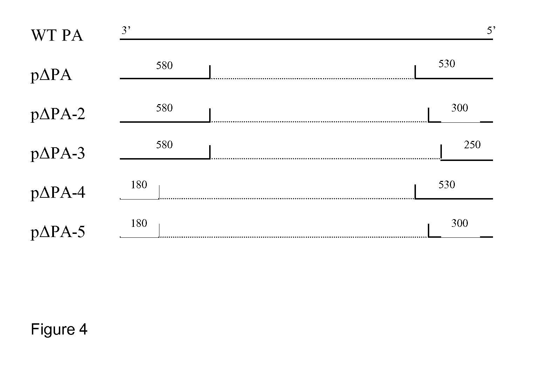

### Legend with figure 4

Additional larger parts of the pΔPA construct that were deleted, resulting in pΔPA-2, pΔPA-3, pΔPA-4, pΔPA-5.

### Detailed description

### Example 1

### Generation of defective influenza A virus particles from recombinant DNA

Influenza A virus is a negative sense, segmented virus. The genome consists of eight gene segments. All eight functional gene segments are required to produce infectious virus, i.e. replicative virus that is capable of unlimited or at least several rounds of replication in cells commonly considered suitable for influenza virus replication. The packaging process of the gene segments of influenza A virus, either through a random or a specific mechanism, has been under debate for many years. Pieces of evidence for both options have been described. Evidence for random packaging is that aggregated virus particles have a higher infectivity than nonaggregated virus particles (6) and that when a cell culture is infected at a low moi, some infected cells lack the expression of one segment (8), both suggesting that there are virions that do not contain the entire influenza virus genome. Further evidence of random packaging is that influenza viruses containing nine segments have been produced experimentally (4). Bancroft and Parslow found that there was no competition between vRNAs originating from the same gene segment for packaging in the virion (1).

One argument for a specific packaging process is that although all gene segments are present in equal amounts in virus stocks, they are present in the producer cells in different amounts (10). Furthermore, when defective interfering (DI) particles are generated, the DI vRNA replaces the segment from which it is derived (3) (A defective interfering particle is a virus particle in which one of the gene segments has a large internal deletion. These particles occur when virus is passaged at a high moi and are also thought to occur due to a R638A mutation of the polymerase acidic protein [Fodor et al; J. Virol. 77, 5017-5020, 2003]). Finally, the efficiency of virion formation increases with an increasing number of gene segments (5). Fujii et al. also showed the region of the NA segment that is required for efficient incorporation of the segment into the virion and later the same group also showed the region of HA and NS important or packaging into the virus particle [Fujii, 2005 #256;Watanabe, 2003 #184].

Here, we present evidence for specific packaging. In order to produce virus particles that contain only seven functional gene segments, we need to determine which gene segment can be left out without abrogating virus production. In the light of the use of a replication deficient virus as a vaccine, HA and NA were not to be left out, and neither were MA or NS because in that case of the need for 2 separate expression plasmids. We produced virus lacking a polymerase gene. We were not able to produce virus when the deleted gene segment was not *trans-*complemented with an expression plasmid (Table 1, 2 and 3, rPR8-7ntc) Virus could be produced upon transfection of seven gene segments and a plasmid expressing the protein normally expressed by the deleted gene segment at very low titers(Table 1, 2 and 3, rPR8-7). Therefore, deletion mutants of gene segments 1,2 and 3 of influenza virus A/WSN/33 were produced harboring an internal deletion of 1032, 528 and 1120 nucleotides, respectively. These deletion mutants were named pΔPB2, pΔPB1 and pΔPA see figure 2). 293T cells were transfected as described previously (de Wit, E., M. I. Spronken, T. M. Bestebroer, G. F. Rimmelzwaan, A. D. Osterhaus, and R. A. Fouchier. 2004. Efficient generation and growth of influenza virus A/PR/8/34 from eight cDNA fragments. Virus Res 103:155-61) with one of each of these deleted gene segments and seven complementing bidirectional constructs encoding A/PR/8/34 (De Wit et al, 2004) and the appropriate expression plasmid. Supernatants were harvested 48h post transfection. Subsequently, MDCK cells were transfected as described previously (2) with one of the expression plasmids HMG-PB2, HMG-PB1 or HMG-PA . These transfected cells were inoculated with the corresponding supernatant of the transfected 293T cells (see figure 1 for explanation of the experimental procedure). Virus replication in these MDCK cells was determined by HA-assay. Initially there was no virus replication in untransfected MDCK cells. Virus replication was shown in MDCK cells transfected either with HMG-PB2, HMG-PB1 or HMG-PA inoculated with the corresponding supernatant. Next, we cloned a defective PA gene segment based on the sequence of a defective interfering PA vRNA of influenza virus A/PR/8/34 obtained from the influenza sequence database (www.flu.lanl.gov, accession number K00867). The 5' 207 nt and 3' 194nt of PA were PCR-amplified and cloned in a bidirectional transcription vector derived from pHW2000 (7) that was modified as described previously (De Wit et al., 2004). The resulting plasmid was called pDIPA, see figure 2). 293T cells were transfected with pDIPA, HMG-PA and 7 bidirectional constructs encoding the remaining gene segments of influenza virus A/PR/8/34 (see figure 2). Supernatant was harvested 48h after transfection and subsequently, MDCK cells transfected with HMG-PA 24h previous, were inoculated with this supernatant. A HA-assay was performed on the supernatant of these MDCK cells 72h after inoculation and was found to be positive, indicating virus replication in these cells. Inoculation of untransfected MDCK cells also did not result in virus production as determined by HA-assay. Subsequent passaging of supernatants containing PA-defective virus particles on MDCK cells either untransfected or transfected with HMG-PA led to the same result (table 1). Up to passage 4, virus was produced in MDCK cells transfected with HMG-PA. The supernatant of MDCKp4 was serially diluted to obtain an indication of virus titer, which was shown to be approximately 10⁴ TCID₅₀/ml..

Method steps used were: 293T cells are transfected (for transfection protocol, see De Wit et al., 2004) with one of the constructs pΔPB2, pΔPB1, pΔPA, pΔNP, the seven complementing constructs encoding A/PR/8/34 (De Wit et al., 2004) and one of HMG-PB2, HMG-PB1, HMG-PA, (expression plasmids are for example described in Pleschka, S., R. Jaskunas, O. G. Engelhardt, T. Zurcher, P. Palese, and A. Garcia-Sastre. 1996. A plasmid-based reverse genetics system for influenza A virus. J Virol 70:4188-92.; obtained from A. Garcia-Sastre and P. Palese). At 48 hours after transfection, the supernatant of the transfected 293T cells is harvested. When viruses were produced, they are present in the supernatant. At the same time, MDCK cells are transfected (for transfection protocol see Basler et al., 2000) with one of the expression plasmids HMG-PB2, HMG-PB1, HMG-PA, (depending on the deletion mutant used, so in the case of using pΔPB2, the MDCK cells are now transfected with HMG-PB2) because the viruses produced lack a gene segment expressing this protein because they have packaged the gene segment with an internal deletion. At 24 hours after transfection, the transfected MDCK cells are inoculated with the supernatant obtained from the transfected 293T cells. When virus is present in the 293T supernatant, this virus will now replicate in the transfected MDCK cells and more virus is produced. This supernatant can again be harvested 72 hours after inoculation.

To prove that no recombination of PA or DIPA with occurred that resulted in a functional PA gene segment, RNA was isolated from the supernatant of MDCKp4. First, the supernatants were passed through a 22µM filter and concentrated by centrifugation. Subsequently, RNA was isolated and a RT-PCR was performed using primers directed to the non-coding regions of the PA segment. RT-PCR performed with primers specific for PA vRNA showed that ΔPA and DIPA remain stable over multiple passaging. In supernatant of MDCK cells infected with DIPA virus particles, a clear band of approximately 400bp appears, in supernatant of MDCK cells infected with virus containing ΔPA, a band of 1100bp appears. In the supernatant of MDCK cells infected with wild type A/PR/8/34 a band of around 2300nt is visible (figure 3). These results indicate that ΔPAPR8 gene segment is stably packaged into virions

To produce viruses lacking PB2, 293T cells were transfected with 7 bi-directional constructs ( Hoffmann, E., G. Neumann, Y. Kawaoka, G. Hobom, and R. G. Webster. 2000. A DNA transfection system for generation of influenza A virus from eight plasmids. Proc Natl Acad Sci U S A 97:6108-13.) encoding gene segments 2, 3, 4, 5, 6, 7 and 8 of influenza virus A/PR/8/34 (de Wit, E., M. I. Spronken, T. M. Bestebroer, G. F. Rimmelzwaan, A. D. Osterhaus, and R. A. Fouchier. 2004. Efficient generation and growth of influenza virus A/PR/8/34 from eight cDNA fragments. Virus Res 103:155-61), resulting in the expression of vRNA and mRNA. A plasmid expressing PB2 of A/PR/8/34, pHMG-PB2 ( Pleschka, S., R. Jaskunas, O. G. Engelhardt, T. Zurcher, P. Palese, and A. Garcia-Sastre. 1996. A plasmid-based reverse genetics system for influenza A virus. J Virol 70:4188-92.) was co-transfected. As a control, only the 7 bi-directional constructs encoding A/PR/8/34 were transfected, omitting pHMG-PB2. The supernatants were harvested 48h after transfection and inoculated in MDCK cells or MDCK cells transfected with pHMG-PB2 (MDCK-PB2) in a 100mm dish 24h earlier. Three days after inoculation, the supernatant of the inoculated MDCK cells was tested for hemagglutinating activity using turkey erythrocytes as an indicator for virus production. No virus was detected in cells inoculated with supernatant of 293T cells transfected with only 7 gene segments, without pHMG-PB2 (rPR8-7ntc, Table 2). The supernatant of MDCK-PB2 cells inoculated with supernatant of 293T cells transfected with 7 gene segments plus pHMG-PB2 was positive. Subsequently, the rPR8-7 supernatant was passaged in MDCK and MDCK-PB2 cells. rPR8-7 replicated in MDCK-PB2 cells, but not in MDCK cells (Table 2). We next generated a 1032nt deletion mutant of gene segment 1 of influenza virus A/WSN/33, resulting in a 344 amino acid deletion (pAPB2, Figure 2). Recombinant virus containing ΔPB2 (rPR8-ΔPB2) was produced as described above (Fig. 1). No virus could be detected in MDCK cells, whereas virus was detected in the MDCK-PB2 cells inoculated with rPR8-ΔPB2. After passaging rPR8-ΔPB2 there was no evidence of virus production in MDCK cells, in contrast to MDCK-PB2 cells (Table 2).

Viruses lacking PB1 were also produced. 293T cells were transfected with 7 bi-directional constructs encoding gene segments 1, 3, 4, 5, 6, 7 and 8 of influenza virus A/PR/8/34, resulting in the expression of vRNA and mRNA. A plasmid expressing PB1 of A/PR/8/34, pHMG-PB1 was co-transfected. As a control, only the 7 bi-directional constructs encoding A/PR/8/34 were transfected, omitting pHMG-PB1. The supernatants were harvested 48h after transfection and inoculated in MDCK cells or MDCK cells transfected with pHMG-PB1 (MDCK-PB1) in a 100mm dish 24h earlier (2) (Fig. 1). Three days after inoculation, the supernatant of the inoculated MDCK cells was tested for hemagglutinating activity using turkey erythrocytes as an indicator for virus production. No virus was detected in cells inoculated with supernatant of 293T cells transfected with only 7 gene segments, without pHMG-PB1 (rPR8-7ntc, Table 3). The supernatant of MDCK-PB1 cells inoculated with supernatant of 293T cells transfected with 7 gene segments plus pHMG-PB1 was positive. Subsequently, the rPR8-7 supernatant was passaged in MDCK and MDCK-PB1 cells. rPR8-7 replicated in MDCK-PB1 cells, but not in MDCK cells (Table 3). We next generated a 528nt deletion mutant of gene segment 2 of influenza virus A/WSN/33, resulting in a 178 amino acid deletion (pΔPB1, Figure 2). Recombinant virus containing ΔPB1 (rPR8-ΔPB1) was produced as described above (Fig. 1). No virus could be detected in MDCK cells, whereas virus was detected in the MDCK-PB1 cells inoculated with rPR8-ΔPB1. After passaging rPRB-ΔPB1 there was no evidence of virus production in MDCK cells, in contrast to MDCK-PB1 cells (Table 3).

We have thus been able to produce viruses lacking segments 1, 2, or 3, by providing pΔPB2, pΔPB1, or pΔPA/pDIPA constructs and *trans-*complementation using RNA polymerase II-driven PB2, PB1 or PA expression plasmids as described above. The conditionally defective viruses described here can only go through one round of replication in cells that are not *trans-*complemented, but can be propagated in *trans-*complementing cell lines. This is the first time defective viruses are produced using reverse genetics that contain only seven functional gene segments and that can undergo one round of replication, or multiple rounds of replication when the defective gene segment is transcomplemented.

The defective viral particles produced in this way are vaccine candidates, since they can go through one round of replication, without producing infectious virus. A result of this single round of replication is that the vaccine induces both a humoral and a cellular immune response. Despite the fact that these defective particles do not replicate in regular cells, for production purposes a large amount of virus can be grown in a cell line that expresses the defective protein. As we have shown, multiple rounds of replication do not affect the genotype of the virus. Besides the use of defective viral particles as vaccine, they are also candidate vectors for gene delivery and for expression of a foreign protein, since a functional gene can be inserted between the 5' and 3' PA, PB2 or PB1 sequences. This was also shown by Watanabe et al. (11), who replaced both HA and NA with foreign genes and could still produce virus.

### Further truncations of pΔPA

Additionally, larger parts of the pΔPA construct that were deleted, resulting in pΔPA-2, pΔPA-3, pΔPA-4, pΔPA-5 (Figure 4). 293T cells were transfected as described previously (De Wit et al., 2004) with one of each of these deleted gene segments and seven complementing bidirectional constructs encoding A/PR/8/34 (De Wit et al, 2004) and an expression plasmid expressing PA. Supernatants were harvested 48h post transfection. Subsequently, MDCK cells were transfected as described previously (Basler, C. F., et al., 2000. Proc Natl Acad Sci U S A 97:12289-94.) with the expression plasmid HMG-PA. These transfected cells and untransfected cells were inoculated with the supernatant of the transfected 293T cells. Virus replication in these MDCK and MDCK-PA cells was determined by HA-assay. There was no virus replication in untransfected MDCK cells. Virus replication was shown in MDCK cells transfected with HMG-PA inoculated with either one of the supernatants. All of the vRNAs resulting from these constructs were thus packaged into virions (Table 4).

**Table 1. Replication of recombinant influenza A/PR/8/34 viruses lacking an intact PA gene segment in MDCK and MDCK-PA cells.**

| Virus | Hemagglutinating activity in supernatant of | | | | | | | | | | Virus titer | |
|---|---|---|---|---|---|---|---|---|---|---|---|---|
| | MDCK | | | | | MDCK-PA | | | | | TCID₅₀/ml | |
| 5 | P1 | P2 | P3 | P4 | | P1 | P2 | P3 | P4 | | P1 | P4 |
| rPR8-7ntc¹ | - | - | - | - | | - | - | - | - | | - | - |
| rPR8-7 | - | - | - | - | | + | + | + | + | | 5.6x10¹ | < 10¹ |
| rPR8-deltaPA - | | - | - | - | | + | + | + | + | | 3.1x10⁵ | 3.1x10⁴ |
| rPR8-DIPA | - | - | - | - | | + | + | + | + | | 3.1x10⁴ | 3.1x10⁴ |
| rPR180-wt | + | + | + | + | | + | + | + | + | | 1.0x10⁷ | ND |

| | | | | | | | | | | | | |
|---|---|---|---|---|---|---|---|---|---|---|---|---|
| ntc: not trans-complemented (no pHMG-PA was transfected in 293T cells) | | | | | | | | | | | | |

**Table 2. Replication of recombinant influenza A/PR/8/34 viruses lacking an intact PB2 gene segment in MDCK and MDCK-PB2 cells.**

| Virus | Hemagglutininating activity in supernatant of | | | | | | | | |
|---|---|---|---|---|---|---|---|---|---|
| | MDCK | | | | | MDCK-PB2 | | | |
| | | p1 | | p2 | | p1 | | p2 | |
| rPR8-7ntc | | - | | - | | - | | - | |
| rPR8-7 | | - | | - | | + | | + | |
| rPR8-pΔPB2 | | - | | - | | + | | + | |
| rPR8 | | + | | + | | + | | + | |

| | | | | | | | | | |
|---|---|---|---|---|---|---|---|---|---|
| ntc: not trans-complemented (no pHMG-PB2 was transfected in 293T cells) | | | | | | | | | |

**Table 3. Replication of recombinant influenza A/PR/8/34 viruses lacking an intact PB1 gene segment in MDCK and MDCK-PB1 cells.**

| Virus | Hemagglutininating activity in supernatant of | | | | | | | | |
|---|---|---|---|---|---|---|---|---|---|
| | MDCK | | | | | MDCK-PB1 | | | |
| | | p1 | | p2 | | p1 | | p2 | |
| rPR8-7ntc | | - | | - | | - | | - | |
| rPR8-7 | | - | | - | | + | | + | |
| rPR8-pΔPB 1 | | - | | - | | + | | + | |
| rPR8 | | + | | + | | + | | + | |

| | | | | | | | | | |
|---|---|---|---|---|---|---|---|---|---|
| ntc: not trans-complemented (no pHMG-PB1 was transfected in 293T cells) | | | | | | | | | |

**Table 4. Replication of recombinant influenza A/PR/8/34 viruses lacking an intact PA gene segment in MDCK-PA and MDCK cells.**

| Virus containing | Virus replication on | |
|---|---|---|
| | MDCK-PA | MDCK |
| pΔPA | + | - |
| pΔPA-2 | + | - |
| pΔPA-3 | + | - |
| pΔPA-4 | + | - |
| pΔPA-5 | + | - |

### Example 2

### Vaccination with defective recombinant virus.

A conditionally defective recombinant virus lacking a functional PA, PB1 or PB2 gene is produced as described herein based on a high-throughput virus backbone (e.g. derived from the vaccine strain A/PR/8/34) with the HA and NA genes of a relevant epidemic virus (e.g. A/Moscow/10/99). The conditionally defective virus is produced by transfection, whereby polymerase protein expression is achieved through trans-complementation. The virus is subsequently amplified in the appropriate cellular substrate such as MDCK cells or Vero cells stably expressing the relevant polymerase. The viral supernatant is cleared by centrifugation for 10 min. at 1000 x g. The virus is concentrated and purified by ultracentrifugation in 20-60 % sucrose gradients, pelleted, and resuspended in phosphate-buffered saline (PBS). Purity and quantity of the virus preparation are confirmed using 12.5 % SDS-polyacrylamide gels stained with coomassie brilliant blue and the virus titer of the conditionally defective virus is determined by infection of MDCK cells and MDCK cells expressing the relevant polymerase and staining with an anti-nucleoprotein monoclonal antibody. Mice are inocculated with 1 x 10E5 50 percent tissue-culture infectious dosis(TCID-50) intratracheal or intra-nasal using a nebulizer. Antibody titers against HA, NA and internal proteins of influenza virus in serum samples collected before and after vaccination are determined using haemagglutination inhibition assays, neuraminidase inhibition assays, ELISA, or virus neutralization assays. The antigen-specific cellular immune response in vaccinated animals is quantified by measuring intracellular cytokine expression by flowcytometry, tetramer-staining of CD4 and CD8-positive cells, cytolytic activity, T-cell proliferation, etc. Vaccinated and control animals are challenged 6 weeks after vaccination using 1 x 10E6 TCID-50 of influenza virus A/Moscow/10/99 or a heterologous virus isolate. After challenge, nasal or pharyngeal swab samples are collected from the animals on a daily basis for 10 days, and the amount of virus excreted by the infected animals are determined by quantitative PCR analyses or virus titrations. The obtained vaccine-induced humoral immunity is detected by quantifying the rise in antibody titers, the obtained vaccine-induced cellular immunity by quantifying the rise in helper and cytotoxic T-cell responses, and the overall level of immunity by confirming protection against infection with a challenge virus.

### References

1. Bancroft, C. T., and T. G. Parslow. 2002. Evidence for segment-nonspecific packaging of the influenza a virus genome. J Virol 76:7133-9.
2. Basler, C. F., X. Wang, E. Muhlberger, V. Volchkov, J. Paragas, H. D. Klenk, A. Garcia-Sastre, and P. Palese. 2000. The Ebola virus VP35 protein functions as a type I IFN antagonist. Proc Natl Acad Sci U S A 97:12289-94.
3. Duhaut, S. D., and J. W. McCauley. 1996. Defective RNAs inhibit the assembly of influenza virus genome segments in a segment-specific manner. Virology 216:326-37.
4. Enami, M., G. Sharma, C. Benham, and P. Palese. 1991. An influenza virus containing nine different RNA segments. Virology 185:291-8.
5. Fujii, Y., H. Goto, T. Watanabe, T. Yoshida, and Y. Kawaoka. 2003. Selective incorporation of influenza virus RNA segments into virions. Proc Natl Acad Sci U S A 100:2002-2007.
6. Hirst, G. K., and M. W. Pons. 1973. Mechanism of influenza recombination. II. Virus aggregation and its effect on plaque formation by so-called noninfective virus. Virology 56:620-31.
7. Hoffmann, E., G. Neumann, Y. Kawaoka, G. Hobom, and R. G. Webster. 2000. A DNA transfection system for generation of influenza A virus from eight plasmids. Proc Natl Acad Sci U S A 97:6108-13.
8. Martin, K., and A. Helenius. 1991. Nuclear transport of influenza virus ribonucleoproteins: the viral matrix protein (M1) promotes export and inhibits import. Cell 67:117-30.
9. Neumann, G., T. Watanabe, and Y. Kawaoka. 2000. Plasmid-driven formation of influenza virus-like particles. J Virol 74:547-51.
10. Smith, G. L., and A. J. Hay. 1982. Replication of the influenza virus genome. Virology 118:96-108.
11. Watanabe, T., S. Watanabe, T. Noda, Y. Fujii, and Y. Kawaoka. 2003. Exploitation of Nucleic Acid Packaging Signals To Generate a Novel Influenza Virus-Based Vector Stably Expressing Two Foreign Genes. J Virol 77:10575-10583.

## Claims

1. A method for obtaining a conditionally defective influenza virus particle comprising a first step of transfecting a suitable first cell or cells,
with one or more gene constructs derived by internally deleting a nucleic acid encoding an influenza protein whereby said gene constructs are incapable of producing a functional protein, but do not hinder packaging of the gene segments of the virus into viral particles
and with complementing influenza virus nucleic acid segments encoding an influenza virus,
and with one or more expression plasmids capable of expressing said proteins in said cell,
and harvesting at least one virus particle from the supernatant of said first cell or cells at a suitable time point after transfection;
and a second step of transfecting a suitable second cell or cells with one or more expression plasmids capable of expressing said proteins in said cell;
and a third step of infecting said second cell or cells with supernatant comprising at least one virus particle obtained from said first cell;
and a fourth step comprising harvesting at least one virus particle from the supernatant of said second cell or cells at a suitable time point after infection.

2. The method according to claim 1, wherein the nucleic acid encoding a nucleoprotein (NP) is not deleted.

3. A method for obtaining a conditionally defective influenza virus particle comprising the step of transfecting a suitable cell or cells,
with one or more gene constructs derived by internally deleting a nucleic acid encoding an influenza polymerase whereby said gene constructs are incapable of producing a functional polymerase, but do not hinder packaging of the gene segments of the virus into viral particles
and with complementing influenza virus nucleic acid segments encoding an influenza virus,
and with one or more expression plasmids capable of expressing said polymerases in said cell,
and harvesting at least one virus particle from the supernatant of said cell or cells at a suitable time point after infection.

4. A method for obtaining a conditionally defective influenza virus particle comprising a first step of transfecting a suitable cell or cells with one or more expression plasmids capable of expressing influenza polymerases in said cell;
and a second step of infecting said cell or cells with supernatant comprising conditionally defective influenza virus particles as defined in claim 3;
and a third step comprising harvesting at least one virus particle from the supernatant of said cell or cells at a suitable time point after infection.

5. A method for obtaining a conditionally defective influenza virus particle comprising a first step of transfecting a suitable first cell or cells,
with one or more gene constructs derived by internally deleting a nucleic acid encoding an influenza polymerase whereby said gene constructs are incapable of producing a functional polymerase, but do not hinder packaging of the gene segments of the virus into viral particles
and with complementing influenza virus nucleic acid segments encoding an influenza virus,
and with one or more expression plasmids capable of expressing said polymerases in said cell,
and harvesting at least one virus particle from the supernatant of said first cell or cells at a suitable time point after transfection;
and a second step of transfecting a suitable second cell or cells with one or more expression plasmids capable of expressing said polymerases in said cell;
and a third step of infecting said second cell or cells with supernatant comprising at least one virus particle obtained from said first cell;
and a fourth step comprising harvesting at least one virus particle from the supernatant of said second cell or cells at a suitable time point after infection.

6. The method according to any one of claims 3 and 5, whereby the internal deletion resulting from internally deleting a nucleic acid encoding an influenza polymerase:
starts at a 5'-nucleotide situated between, but not encompassing, nucleotides 58 and 207 counted from the non-coding region, and finishes at a 3'-nucleotide situated between, but not encompassing, nucleotides 27 and 194 counted from the non-coding region for the PA protein,
starts at a 5'-nucleotide situated between, but not encompassing, nucleotides 43 and 246 counted from the non-coding region, and finishes at a 3'-nucleotide situated between, but not encompassing, nucleotides 24 and 197 counted from the non-coding region for the PB1 protein,
starts at a 5'-nucleotide situated between, but not encompassing, nucleotides 34 and 234 counted from the non-coding region, and finishes at a 3'- nucleotide situated between, but not encompassing, nucleotides 27 and 209 counted from the non-coding region for the PB2 protein.

7. The method according to any one of claims 4-6, whereby the cell or cells to be infected with supernatant comprising conditionally defective influenza virus particles already express the non-functional polymerases.

8. A method according to any one of claims 3-7 wherein said polymerase is acidic polymerase (PA).

9. A method according to any one of claims 3-7 wherein said polymerase is basic polymerase 1 (PB1).

10. A method according to any one of claims 3-7 wherein said polymerase is basic polymerase 2 (PB2).

11. An influenza virus particle obtainable by a method according to anyone of claims 1 to 10.

12. A conditionally defective influenza virus particle comprising one or more nucleic acid segments with an internal deletion in the segment rendering the segment incapable of producing a functional influenza polymerase, but not hindering packaging of the gene segment of the virus into viral particles, whereby the polymerase is selected from the group of acidic polymerase (PA), basic polymerase 1 (PB1) or basic polymerase 2 (PB2).

13. An influenza virus particle comprising one or more nucleic acid segments with an internal deletion in the segment rendering the segment incapable of producing a functional influenza polymerase, but not hindering packaging of the gene segment of the virus into viral particles, whereby the polymerase is selected from the group of acidic polymerase (PA), basic polymerase 1 (PB1) or basic polymerase 2 (PB2), and whereby the internal deletion:
starts at a 5'-nucleotide situated between, but not encompassing, nucleotides 58 and 207 counted from the non-coding region, and finishes at a 3'- nucleotide situated between, but not encompassing, nucleotides 27 and 194 counted from the non-coding region for the PA protein,
starts at a 5'-nucleotide situated between, but not encompassing, nucleotides 43 and 246 counted from the non-coding region, and finishes at a 3'- nucleotide situated between, but not encompassing, nucleotides 24 and 197 counted from the non-coding region for the PB1 protein,
starts at a 5'-nucleotide situated between, but not encompassing, nucleotides 34 and 234 counted from the non-coding region, and finishes at a 3'- nucleotide situated between, but not encompassing, nucleotides 27 and 209 counted from the non-coding region for the PB2 protein.

14. A particle according to anyone of claims 11-13 having the influenza virus nucleic acid segments encoding the viral glycoproteins.

15. A particle according to anyone of claims 11 to 14 having the influenza virus nucleic acid segments encoding the nucleoprotein (NP), the basic polymerase 1 (PB1), the basic polymerase 2 (PB2), the haemagglutinin (HA), the neuraminidase (NA), the matrix proteins (M1 and M2) and the nonstructural protein (NS1 and NS2).

16. A particle according to anyone of claims 11 to 14 having the influenza virus nucleic acid segments encoding the nucleoprotein (NP), the acid polymerase (PA), the basic polymerase 2 (PB2), the haemagglutinin (HA), the neuraminidase (NA), the matrix proteins (M1 and M2) and the nonstructural protein (NS1 and NS2).

17. A particle according to anyone of claims 11 to 14 having the influenza virus nucleic acid segments encoding the nucleoprotein (NP), the acid polymerase (PA), the basic polymerase 1 (PB1), the haemagglutinin (HA), the neuraminidase (NA), the matrix proteins (M1 and M2) and the nonstructural protein (NS1 and NS2).

18. A particle according to anyone of claims 11 to 17 having influenza virus nucleic acid segments that are derived from influenza A virus.

19. A particle according to anyone of claims 11 to 18 provided with a foreign nucleic acid encoding a foreign gene or gene fragment.

20. A cell comprising a particle according to anyone of claims 11 to 19.

21. A cell according to claim 20 having been provided with one or more influenza virus polymerases, whereby the polymerase is selected from the group of acidic polymerase (PA), basic polymerase 1 (PB1) or basic polymerase 2 (PB2).

22. A composition comprising a particle according to anyone of claims 11 to 19 or a cell or material derived from a cell according to claim 20 or 21.

23. The composition according to claim 22 for use in the generation of immunological protection against infection of a subject with an influenza virus.

24. Use of a composition according to claim 22 for the production of a pharmaceutical composition directed at generating immunological protection against infection of a subject with an influenza virus.

25. Use of a particle according to claim 19 for the production of a composition directed at delivery of a foreign nucleic acid encoding a foreign gene or gene fragment to a cell.

26. Use of a particle according to claim 19 for the production of a pharmaceutical composition directed at delivery of a foreign nucleic acid encoding a foreign gene or gene fragment to a subject's cells.

27. A method for delivery of a foreign nucleic acid encoding a foreign gene or gene fragment to a cell comprising providing said cell with a defective influenza virus particle according to claim 19.

28. The defective influenza virus particle according to claim 19 for use in delivering a foreign nucleic acid encoding a foreign gene or gene fragment to a subject.
